# EUROPÄISCHE PATENTANMELDUNG

(11) **EP 1 174 086 A1**
(43) Veröffentlichungstag der Anmeldung: **23.01.2002**
(21) Anmeldenummer: 01000301.0
(22) Anmeldetag: 17.07.2001
(51) Int. Cl.: A61B 6/03, G06T 11/00

(54) **Computertomographie-Verfahren mit kegelförmiger Durchstrahlung eines Objekts**

(30) Priorität: 19.07.2000 DE 10035138
(71) Anmelder: Philips Corporate Intellectual Property GmbH, 52064 Aachen (DE); Koninklijke Philips Electronics N.V., 5621 BA Eindhoven (NL)
(72) Erfinder: Köhler, Thomas, Habsburgerallee 11 52064, Aachen (DE); Grass, Michael, Habsburgerallee 11 52064, Aachen (DE)
(74) Vertreter: Volmer, Georg, Dipl.-Ing.

(57) **Zusammenfassung**

Die Erfindung betrifft ein Computertomographie-Verfahren, bei dem mit einem kegelförmigen Strahlenbündel fortlaufend eine Folge von CT-Datensätzen entlang einer geschlossenen, vorzugsweise kreisförmigen Trajektorie ermittelt wird. Da ein entlang einer solchen Trajektorie ermittelter CT-Datensatz unvollständig ist, gibt es Artefakte, die bei der Erfindung durch die folgenden Schritte verringert werden:
- Akquisition wenigstens eines ersten, vollständigen CT-Datensatzes,
- Akquisition einer Folge von zweiten CT-Datensätzen, wobei die Relativbewegung zwischen dem Strahlenbündel und dem Objekt jeweils einer geschlossenen - vorzugsweise kreisförmigen - Bahn entspricht,
- Ergänzung der Daten der zweiten Datensätze durch Daten des ersten Datensatzes Rekonstruktion einer Folge von CT-Bildern, aus den ergänzten zweiten CT-Datensätzen

## Beschreibung

Die Erfindung betrifft ein Computertomographie-Verfahren, bei dem ein kegelförmiges Strahlenbündel ein Objekt durchsetzt und bei dem fortlaufend CT-Daten (CT= Computertomographie) akquiriert und CT-Bilder rekonstruiert werden. Bei solchen Anwendungen können keine helixförmigen Trajektorien (als Trajektorie wird die Relativbewegung zwischen dem Strahlenbündel und dem Objekt bezeichnet) benutzt werden, weil diese eine Umkehr der Bewegungsrichtung voraussetzen, was zu Bewegungsunschärfen in den rekonstruierten CT-Bildern führen würde.

Für derartige Anwendungen benutzt man daher geschlossene, vorzugsweise ebene, insbesondere kreisförmige Trajektorien. Es ist aber bekannt, dass man mit einer kreisförmigen Trajektorie nur für die Schicht vollständige Daten hat, die in der Ebene der Trajektorie liegt. Aufgrund dieses sogenannten "missing-data" Problems ergeben sich daher in den rekonstruierten Bildern Artefakte, die um so ausgeprägter sind, je größer der Winkel ist, den die Strahlen zu den rekonstruierenden Voxeln mit der Ebene der Trakjektorie einschließen.

Aufgabe der vorliegenden Erfindung ist es, ein Computertompgraphie-Verfahren so auszugestalten, dass sich weniger ausgeprägte Artefakte ergeben. Diese Aufgabe wird erfindungsgemäß gelöst durch ein Computertomographie-Verfahren, bei dem ein kegelförmiges Strahlenbündel ein Objekt durchsetzt mit folgenden Schritten:
- Akquisition wenigstens eines ersten, vollständigen CT-Datensatzes,
- Akquisition einer Folge von zweiten CT-Datensätzen, wobei die Relativbewegung zwischen dem Strahlenbündel und dem Objekt jeweils einer geschlossenen - vorzugsweise kreisförmigen - Bahn entspricht,
- Ergänzung der Daten der zweiten Datensätze durch Daten des ersten Datensatzes
- Rekonstruktion einer Folge von CT-Bildern, aus den ergänzten zweiten CT-Datensätzen.
Bei der Erfindung wird also zunächst ein erster vollständiger CT-Datensatz akquiriert. Diese Akquisition erfolgt zweckmäßigerweise unmittelbar vor der eigentlichen "fluoroskopischen" CT-Untersuchung. Danach werden fortlaufend entlang einer zweiten, vorzugsweisen kreisförmigen Trajektorie zweite CT-Datensätze akquiriert. Wie erläutert, sind diese Datensätze unvollständig, was zu den eingangs geschilderten Artefakten bei der Rekonstruktion von CT-Bildern aus diesen zweiten Datensätzen führen würde. Vor der Rekonstruktion werden die Daten des jeweiligen zweiten CT-Datensatzes jedoch durch Daten des ersten CT-Datensatzes ergänzt.

Da dieser erste CT-Datensatz definitionsgemäß vollständig ist, führt die Rekonstruktion aus den ergänzten Daten zu CT-Bildern, die frei von den erwähnten Artefakten sind, wenn es in dem Objektbereich, dem der zweite Datensatz zugeordnet ist, keine Veränderungen gegeben hat (gegenüber dem Zeitpunkt, an dem der erste Datensatz akquiriert worden ist). Wenn sich in diesem Bereich Veränderungen ergeben haben - z.B. durch einströmendes Kontrastmittel, durch Einführen eines chirurgischen Instruments oder durch Objektbewegungen -, dann machen sich die erwähnten Artefakte zwar auch bemerkbar, jedoch weniger ausgeprägt, weil die unverändert gebliebenen Bereiche des Objektes diese Artefakte nicht mehr beeinflussen können.

Es sei an dieser Stelle erwähnt, dass aus der EP-OS 0860144A2 bereits ein Computertomographie-Verfahren bekannt ist, bei dem zunächst ein erster CT-Datensatz und danach eine Folge von zweiten CT-Datensätzen akquiriert wird. Der erste Datensatz wird dabei entlang einer helixförmigen Trajektorie akquiriert; er ist deshalb vollständig. Über die Trajektorie, entlang der die zweiten Datensätze akquiriert werden, enthält die EP-OS 0860144A2 keine näheren Angaben, so dass unklar ist, ob der zweite Datensatz vollständig oder unvollständig ist.

Jedoch werden aus den CT-Datensätzen getrennt CT-Bilder rekonstruiert. Aus dem ersten Datensatz wird ein CT-Bild rekonstruiert, das die Anatomie des Patienten darstellen soll, und aus dem zweiten Datensatz ein CT-Bild, das bei beispielsweise ein in den Patienten eingeführtes Instrument darstellen soll. Die beiden CT-Bilder werden miteinander kombiniert, so dass man die Lage des chirurgischen Instruments innerhalb des Patienten erkennen kann.

Das zweite, lediglich aus den Daten des zweiten CT-Datensatzes rekonstruierte CT-Bild enthält die aufgrund des "missing-data" Problems resultierenden Artefakte daher in voller Höhe. Wenn das zweite CT-Bild darüber hinaus noch so rekonstruiert wird, dass die darin im Prinzip enthaltene anatomische Information unterdrückt wird, dann werden Veränderungen in dem anatomischen Bereich, z.B. aufgrund einer Verschiebung des Patienten, in dem Kombinationsbild nicht erkennbar. Das kann fatale Folgen haben, wenn mit dem chirurgischen Instrument ein Tumor entfernt werden soll, dessen Lage sich zwischen der Akquisition des ersten Datensatzes und des zweiten Datensatzes verändert hat.

Bei der Erfindung sind demgegenüber die Artefakte verringert, und alle Veränderungen (nicht nur die durch die Einführung z.B. eines Instruments erfolgenden) in dem abgebildeten Objektbereich werden zutreffend dargestellt. Der zwite Datensatz kann auf verschiedene Weise durch die Daten des ersten Datensatzes ergänzt werden, bevor daraus ein CT-Bild rekonstruiert wird:

Eine erste, bevorzugte Lösung ist in Anspruch 2 angegeben. Dabei wird zunächst das erste CT-Bild rekonstruiert. Danach ist die sogenannte "Objektfunktion" bekannt, d.h. die räumliche Verteilung der Schwächung der Strahlung innerhalb des Objektes. Man kann daher aus dem ersten CT-Bild virtuelle CT-Daten berechnen für die der Akquisition des zweiten Datensatzes zugrundeliegende Trajektorie. Wenn sich in dem Objektbereich, der vom zweiten Datensatz erfaßt wird, keine Veränderungen ergeben, müssen diese virtuellen Daten identisch sein mit den CT-Daten des zweiten CT-Datensatzes. Die Rekonstruktion eines CT-Bildes aus der Differenz der jeweils für dieselben Strahlen geltenden virtuellen CT-Daten und der CT-Daten des zweiten Datensatzes liefert daher ein Differnz-CT-Bild, das lediglich die Veränderungen in dem Objektbereich darstellt.

Ein solches CT-Bild macht die Orientierung in vielen Fällen schwer. Wenn man hingegen gemäß Anspruch 3 dem Differenz-CT-Bild das erste CT-Bild -mit geeigneter Gewichtung - überlagert, dann ist eine Orientierung in dem Überlagerungsbild ohne weiteres möglich. Eine zweite Möglichkeit zur Ergänzung der CT-Daten des zweiten CT-Datensatzes durch Daten des ersten CT-Datensatzes vor der Rekonstruktion ist in Anspruch 4 angegeben. Bekanntlich kann man die entlang einer Trajektorie akquirierten CT-Daten im sogenannten Radon-Raum beschreiben. Bei einer kreisförmigen Trajektorie füllen die berechenbaren Radon-Werte im Radon-Raum einen ringförmigen Torus. Für eine vollständige Rekonstruktion müßten jedoch die Werte in einem Rotationselllipsoid bzw. in einem kugelförmigen Bereich des Radon-Raums bekannt sein. Dieser für eine vollständige Rekonstruktion erforderliche Bereich des Radon-Raums ergibt sich, wenn man die aus dem zweiten Datensatz nicht berechenbaren und für eine vollständige Rekonstruktion erforderlichen Radon-Werte aus Radon-Werten des ersten Datensatzes ergänzt, bevor man aus diesen Radon-Werten ein CT-Bild rekonstruiert.

Eine bevorzugte Möglichkeit für die Akquisition des ersten Datensatzes ist in Anspruch 5 angegeben. Zwar kann man einen vollständigen Datensatz auch entlang anderer Trajektorien erhalten (z.B. entlang zweier sich schneidender kreisförmiger Trajektorien), doch ist die Erzeugung eine helixförmigen Relativbewegung besonders einfach, und es sind dafür geeignete Rekonstruktionsverfahren bekannt.

Anspruch 6 definiert einen zur Durchführung des Verfahrens nach Anspruch 1 geeigneten Computertomographen, und Anspruch 7 beinhaltet ein Computerprogramm, mit dem sich die Steuereinheit eines Computertomographen so steuern läßt, dass das erfindungsgemäße Computertomographie-Verfahren ausgeführt wird.

Die Erfindung wird nachstehend anhand der Zeichnungen näher erläutert. Es zeigen:
Fig. 1 einen Computertomographen, mit dem die Erfindung ausführbar ist,
Fig. 2 das Ablaufdiagramm einer ersten Ausführungsform der Erfindung,
Fig. 3 die Darstellung der einander ergänzenden Radon-Werte im Radon-Raum,
Fig. 4 das Ablaufdiagramm einer zweiten Ausführungsform des erfindungsgemäßen Verfahrens und
Fig. 5 eine die Berechnung der virtuellen CT-Daten erläuternde Darstellung.

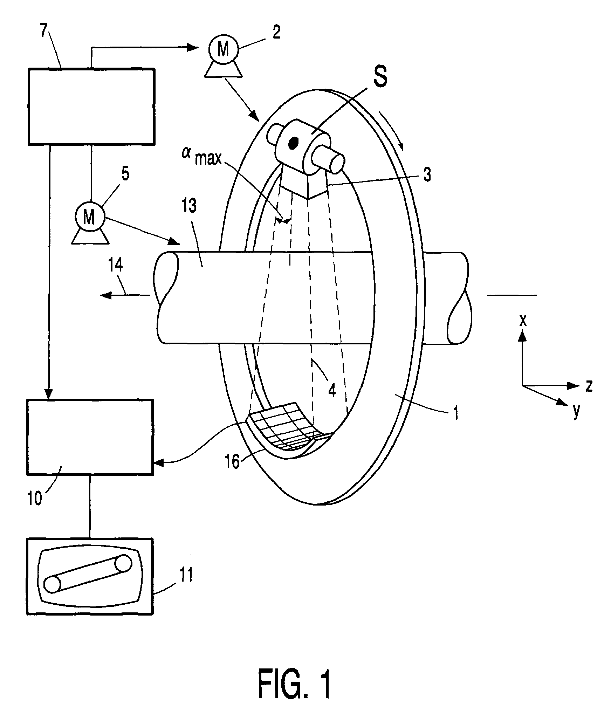

Der in Fig. 1 dargsstellte Computertomograph umfaßt eine Gantry 1, die um eine parallel zur z-Richtung des in Fig. 1 dargestellten Koordinatensystems verlaufende Rotationsachse 14 rotieren kann. Dazu wird die Gantry von einem Motor 2 mit einer vorzugsweisen konstanten, aber einstellbaren Winkelgeschwindigkeit angetrieben. An der Gantry ist eine Strahlenquelle S befestigt, beispielsweise ein Röntgenstrahler. Dieser ist mit einer Kollimatoranordnung 3 versehen, die aus der von der Strahlenquelle S erzeugten Strahlung ein kegelförmiges Strahlenbündel 4 ausblendet, d.h. ein Strahlenbündel, das sowohl in z-Richtung als auch in einer dazu senkrechten Richtung (d.h. in einer zur Rotationsachse senkrechten Ebene) eine von Null verschiedene endliche Ausdehnung hat.

Das Strahlenbündel 4 durchdringt einen Untersuchungsbereich 13, in dem sich ein Objekt, z.B. ein Patient auf einem Patientenlagerungstisch (beides nicht näher dargestellt), befinden kann. Der Untersuchungsbereich 13 hat die Form eines Zylinders. Nach dem Durchsetzen des Untersuchungsbereichs 13 trifft das Röntgenstrahlenbündel 4 auf eine an der Gantry 1 befestigte zweidimensionale Detektoreinheit 16, die eine Anzahl von nebeneinander angeordneten Detektorzeilen mit jeweils einer Vielzahl von Detektorelementen umfaßt. Die Detektorzeilen befinden sich in zur Rotationsachse 14 senkrechten Ebenen, vorzugsweise auf einem Kreisbogen um die Strahlenquelle S. Sie können aber auch anders geformt sein, z.B. einen Kreisbogen um die Rotationsachse 14 beschreiben oder geradlinig rein. Jedes von dem Strahlenbündel 4 getroffene Detektorelement liefert in jeder Position der Strahlenquelle einen Meßwert für einen Strahl aus dem Strahlenbündel 4. Die Meßwerte werden im folgenden auch als CT-Daten bezeichnet.

Der mit αₘₐₓ bezeichnete Öffnungswinkel des Strahlenbündels 4 (als Öffnungswinkel ist der Winkel definiert, den ein Strahl, der in einer zur Rotationsachse 14 senkrechten Ebene am Rande des Strahlenbündels 4 liegt, mit einer durch die Strahlenquelle S und die Rotationsachse 14 definierten Ebene einschließt) bestimmt dabei den Durchmesser des zylinderförmigen Untersuchungsbereiches 13, innerhalb dessen sich das zu untersuchende Objekt bei der Akquisition der CT-Daten befinden muß.

Der Untersuchungsbereich 13- bzw. das Objekt oder der Patientenlagerungstisch - kann mittels eines Motors 5 parallel zur Rotationsachse 14 bzw. parallel zur z-Achse verschoben werden. Dazu äquivalent könnte aber auch die Gantry in diese Richtung verschoben werden; wesentlich ist allein die Relativverschiebung. Wenn die Motoren 5 und 2 gleichzeitig laufen, beschreiben die Strahlenquelle S und die Detektoreinheit 16 eine helixförmige Trajektorie relativ zum Untersuchungsbereich 13. Wenn hingegen der Motor 5 für den Vorschub in z-Richtung stillsteht und der Motor 2 die Gantry 1 rotieren läßt, ergibt sich eine kreisförmigs Trajektorie für die Strahlenquelle S und die Detektoreinheit 16 relativ zum Untersuchungsbereich 13.

Die von der Detektoreinheit 16 akquirierten Meßwerte bzw. CT-Daten werden einem Bildverarbeitungsrechner 10 zugeführt, der daraus die Absorptionsverteilung in einem Teil des Untersuchungsbereichs 13 rekonstruiert und z.B. auf einem Monitor 11 wiedergibt. Die beiden Motoren 2 und 5, der Bildverarbeitungsrechner 10, die Strahlenquelle S und der Transfer der CT-Daten von der Detektoreinheit 16 zum Bildverarbeitungsrechner 10 werden von einer Steuereinheit 7 gesteuert.

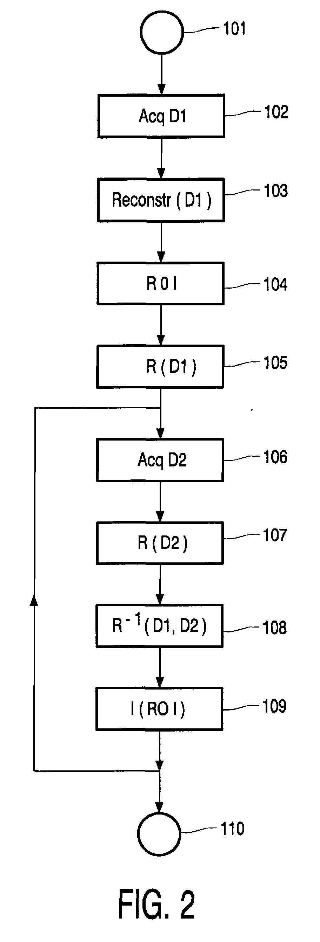

Fig. 2 zeigt den Ablauf eines CT-Verfahrens, das mit dem Computertomographen nach Fig. 1 durchgeführt werden kann. Nach der Initialisierung im Block 101 werden die Motoren 2 und 5 gleichzeitig eingeschaltet, so dass die Strahlenquelle S bzw. die Detektoreinheit 16 relativ zum Untersuchungsbereich bzw. zu dem darin befindlichen Objekt eine helixförmige Bahn beschreibt. Bei dieser Relativbewegung ist die Strahlenquelle S eingeschaltet, und die dabei akquirierten Meßwerte werden in einem Speicher des Bildverarbeitungsrechners 10 gespeichert.

Danach wird im Schritt 103 aus dem auf diese Weise gewonnenen ersten CT-Datensatz ein erstes CT-Bild rekonstruiert. Ein geeignetes Rekonstruktionsverfahren ist beispielsweise aus der US-Appln.09/368850 (PHD 98-086) bekannt.

Im nächsten Schritt 104 wird der für die Untersuchung relevante Bereich des Objektes (englisch: region of interest, ROI) festgelegt, der bei der weiteren Untersuchung fortlaufend dargestellt werden soll. Die Gantry wird dann parallel zur Rotationsachse verschoben, bis die gewünschte Region des Objektes bei einer kreisförmigen Trajektorie (d.h. wenn Strahlenquelle und Objekt relativ zueinander um die Rotationsachse rotieren, aber nicht in Richtung der Rotationsachse verschoben werden) vom Strahlenbündel 4 erfaßt und rekonstruiert werden kann.

Im Schritt 105 werden dann aus dem ersten CT-Datensatz diejenigen Radon-Werte berechnet, die der Akquisition eines zweiten Datensatzes entlang der zuvor bestimmten Trajektorie fehlen werden, weil sie im Radon-Raum außerhalb des ringförmigen Torus liegen, in dem sich aus dem zweiten Datensatz Radon-Werte ergeben.

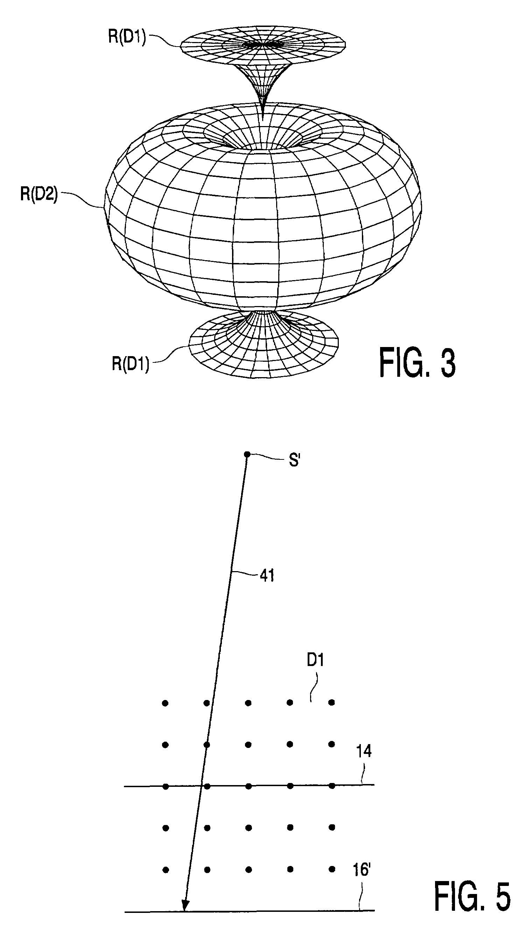

Dies wird in Fig 3 erläutert, die den ringförmigen Torus R(D2) darstellt, der die Punkte im Radon-Raum umschließt, für die sich aus dem zweiten CT-Datensatz Radon-Werte berechnen lassen. Dabei ist angenommen, dass die Rotationsachse in Fig. 3 vertikal verläuft. Oberhalb und unterhalb des Torus R(D2) - und zwar auf dessen Symmetrieachse - liegen die Punkte R(D1) im Radon-Raum, die den ringförmigen Torus zu einer Kugel ergänzen (zu welchem Zweck die vom Torus abgewandten Flächen Kalottenform haben müssen und nicht eben sein dürfen wie in Fig 3 zwecks einfacherer Darstellung angenommen). Für die Punkte in den Bereichen R (D 1) des Radon-Raums werden im Schritt 105 die Radon-Werte berechnet. Ein Radon-Wert für einen Punkt im Radon-Raum berechnet sich bekanntlich als das Flächenintegral der Schwächung der Strahlung in einer diesen Punkt enthaltenden Ebene, die senkrecht zu der Verbindungslinie dieses Punktes mit dem Null-Punkt des Radon-Raumes verläuft.

Im Schritt 106 wird nur der Motor 2 eingeschaltet, so dass die Strahlenquelle und die Detektoreinheit 16 um die Rotationsachse 14 und damit um den Untersuchungsbereich 13 bzw. das darin befindliche Objekt rotieren. Dabei wird die Strahlung der Strahlenquelle S eingeschaltet, und die von den Detektorelementen der Detektoreinheit 16 akquirierten CT-Daten werden erfaßt und gespeichert. Die Gesamtheit dieser bei einem Umlauf der Strahlenquelle akquirierten Daten stellt einen zweiten CT-Datensatz dar.

Aus diesem zweiten Datensatz werden im Schritt 107 die Radon-Werte für alle Punkte innerhalb des ringförmigen Torus R(D2) - vgl. Fig. 3 - berechnet. Nachdem die Radon-Werte für die Punkte im Radon-Raum berechnet worden sind, die für eine vollständige Rekonstruktion erforderlich sind, erfolgt im Schritt 108 eine inverse Radon-Transformation über diese aus dem zweiten und teilweise aus dem ersten Datensatz abgeleiteten Radon-Werte. Diese inverse Radon-Transformation ist bekannt, beispielweise aus dem Buch von F. Natterer "The mathematics of computerized tomography" (ISBN 3 519 02103 X), Kapitel II.2).

Nach diesem Rekonstruktionsschritt ergibt sich ein (dreidimensionales) CT-Bild, das die Objektfunktion bzw. die Schwächung der Strahlung im Untersuchungsbereich als Funktion des Ortes darstellt. Dieses CT-Bild wird im Schritt 109 auf dem Monitor 11 in geeigneter Weise dargestellt, z.B. in Form eines Schichtbildes für eine in dem dreidimensionalen Bereich enthaltene Schicht.

Danach wird die aus den Schritten 106 bis 109 bestehende Schleife erneut durchlaufen, um aus den dabei akquirierten Daten (ergänzt durch die Radon-Werten R(D1)) ein weiteres CT-Bild zu rekonstruieren. Die Akquisition der Daten könnte dabei schon begonnen werden, bevor die Rekonstruktion und/oder die Wiedergabe des CT-Bildes in den Schritten 108 und 109 beendet ist. Wenn die Untersuchung beendet ist, endet das Verfahren im Schritt 110.

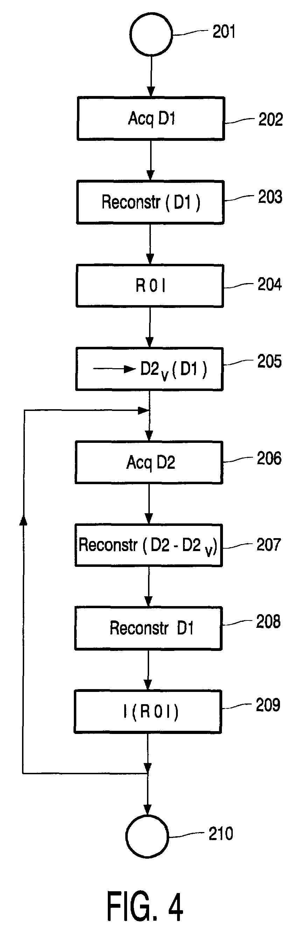

Im folgenden wird eine weitere, bevorzugte Ausführungsform der Erfindung anhand der Fig. 4 und 5 erläutert. Die Schritte 201 bis 204 entsprechen dabei den Schritten 101 bis 104 in dem anhand der Fig. 2 und 3 erläuterten Verfahren. Im Schritt 205 erfolgt eine Berechnung von virtuellen CT-Daten für eine (bei der nachfolgende Akquisition der zweiten Datensätze benutzte) kreisförmige Trajektorie aus dem im Schritt 203 rekonstruierten ersten CT-Bild.

Zur Erläuterung dieses Schrittes wird auf Fig. 5 verwiesen, wo durch jeweils einen Punkt die Voxel symbolisiert sind, für die die Objektfunktion bzw. die Schwächung der Strahlung in Form des ersten CT-Bildes ermittelt worden ist. Diese Punkte definieren ein regelmäßiges, vorzugsweise kartesisches, dreidimensionales Gitter (oberhalb und unterhalb der Zeichenebene der Fig 5 gibt es weitere Voxel). Außerdem ist eine der Positionen S' dargestellt, die die Strahlenquelle S bei der Akquisition weiterer Datensätze auf einer kreisförmigen Trajektorie relativ zu dem rekonstruierten Teil des Untersuchungsbereiches einnimmt. Schließlich ist auch noch die zugehörige Position 16' der Detektoreinheit 16 dargestellt.

Verbindet man die Position S' mit einem Detektorelement der in der Position 16' befindlichen Detektoreinheit durch eine Gerade bzw. einen Strahl 41, dann läßt sich die Schwächung der Strahlung entlang des Strahles 41 aus den für die verschiedenen Voxel in dem ersten Datensatz ermittelten Schwächungswerte berechnen. Führt man diese Berechnung für alle Detektorelemente der Detektoreinheit 16 und für jede Position der Strahlenquelle auf der kreisförmigen Trajektorie durch, dann erhält man einen Satz virtueller CT-Daten, der zu dem im anschließenden Schritt 206 (der dem Schritt 106 in dem anhand der Fig. 2 und 3 erläuterten Verfahren entspricht) entlang einer kreisförmigen Trajektorie akquirierten zweiten CT-Datensatz korrespondiert.

Wenn zwischen der Akquisition des ersten und des zweiten Datensatzes in dem durch den zweiten Datensatz abgebildeten Bereich keine Veränderung stattgefunden hat (und wenn die Rekonstruktion des ersten CT-Bildes aus dem ersten Datensatz und die Berechnung der virtuellen CT-Daten aus dem ersten CT-Bild ganz exakt ist), dann entsprechen für sämtliche Strahlen die zugehörigen CT-Daten des zweiten Datensatzes und die virtuellen Daten einander. Wenn sich jedoch eine Veränderung ergeben hat - beispielsweise dadurch, dass in dem abgebildeten Bereich ein chirurgisches Instrument (etwa eine Biopsienadel) eingeführt wird oder dadurch, dass Kontrastmittel in diesen Bereich einströmt oder dadurch, dass sich zumindest stellenweise eine Verschiebung des Objektes zwischen der Akquisition der beiden Datensätze ergeben hat - dann unterscheiden sich die virtuellen CT-Daten von den CT-Daten des zweiten Datensatzes.

Im Schritt 207 werden diese Differenzen zwischen den CT-Daten des zweiten Datensatzes und den virtuellen CT-Daten (jeweils für den gleichen Strahl) zur Rekonstruktion eines Differenz-CT-Bildes herangezogen, das die Veränderungen in den durch den zweiten CT-Datensatz erfaßten Bereich gegenüber dem Zeitpunkt darstellt, in dem der erste CT-Datensatz akquiriert wurde. Die Rekonstruktion erfolgt dabei mit Hilfe der sogenannten gefilterten Rückprojektion (englisch: filtered back-projektion). Ein dafür geeignetes Rekonstruktionsverfahren ist aus der US-Appln 09 /400763 (PHD 98-111) bekannt.
In der Regel ist die anatomische Orientierung in einem solchen Differenz-CT-Bild relativ schwer, und deshalb wird in einem weiteren Rekonstruktionsschritt 208 das Differenz-CT-Bild dem aus dem ersten CT-Datensatz rekonstruierten ersten CT-Bild mit einem geeigneten Gewichtungsfaktor überlagert. Das Überlagerungsbild wird im Schritt 209 dargestellt. Danach werden die Schritte 206 bis 209 so oft wiederholt wie nötig, um dann beendet zu werden (Schritt 210).

## Patentansprüche

1. Computertomographie-Verfahren, bei dem ein kegelförmiges Strahlenbündel ein Objekt durchsetzt mit folgenden Schritten:
- Akquisition wenigstens eines ersten, vollständigen CT-Datensatzes,
- Akquisition einer Folge von zweiten CT-Datensätzen, wobei die Relativbewegung zwischen dem Strahlenbündel und dem Objekt jeweils einer geschlossenen - vorzugsweise kreisförmigen - Bahn entspricht,
- Ergänzung der Daten der zweiten Datensätze durch Daten des ersten Datensatzes
- Rekonstruktion einer Folge von CT-Bildern, aus den ergänzten zweiten CT-Datensätzen.

2. Computertomographie-Verfahren nach Anspruch 1,
**gekennzeichnet durch** folgende Schritte:
- Rekonstruktion eines ersten CT-Bildes aus dem ersten CT-Datensatz,
- Berechnung von virtuellen CT-Daten für die bei der Akquisition eines der zweiten Datensätze das Objekt durchdringenden Strahlen aus dem ersten CT-Bild,
- Berechnung der Differenzen zwischen den virtuellen CT-Daten und den für dieselben Strahlen akquirierten CT-Daten des zweiten CT-Datensatzes
- Rekonstruktion eines Differenz-CT-Bildes aus den Differenzen.

3. Computertomographie-Verfahren nach Anspruch 2,
**gekennzeichnet durch**
die Überlagerung des ersten CT-Bildes und des Differenz-CT-Bildes.

4. Computertomographie-Verfahren nach Anspruch 1,
**gekennzeichnet durch** folgende Schritte
- Berechnung der aus dem zweiten Datensatz nicht berechenbaren Radon-Werte aus dem ersten CT-Datensatz
- Berechnung der aus dem jeweiligen zweiten Datensatz berechenbaren Radon-Werte
- Inverse Radon-Transformation der aus den beiden Datensätzen ermittelten Radon-Werte

5. Computertomographie-Verfahren nach Anspruch 1,
**gekennzeichnet durch**
eine entlang einer helixförmigen Bahn erfolgende Relativbewegung zwischen dem Strahlenbündel und dem Objekt bei der Akquisition des ersten CT-Datensatzes.

6. Computertomograph zur Durchführung des Verfahrens nach Anspruchs 1mit
- einer Strahlenquelle zur Erzeugung eines kegelförmigen Strahlenbündels,
- einer damit gekoppelten Detektoreinheit zur Akquisition von CT-Daten,
- einer Antriebsanordnung, um ein in einem Untersuchungsbereich enthaltenes Objekt und die Strahlenquelle relativ zueinander um eine Rotationsachse rotieren und/oder sich parallel zur Rotationsachse verschieben zu lassen,
- einer Rekonstruktionseinheit zur Rekonstruktion der räumlichen Verteilung der Absorption innerhalb des Untersuchungsbereiches aus den von der Detektoreinheit akquirierten Meßdaten, und
- einer Steuereinheit zur Steuerung der Strahlenquelle, der Detektoreinheit, der Antriebsanordnung und der Rekonstruktionseinheit
**gekennzeichnet durch**
eine Programmierung der Steuereinheit gemäß folgendem Ablauf:
- Akquisition wenigstens eines ersten, vollständigen CT-Datensatzes,
- Akquisition einer Folge von zweiten CT-Datensätzen, wobei die Relativbewegung zwischen dem Strahlenbündel und dem Objekt jeweils einer geschlossenen - vorzugsweise kreisförmigen - Bahn entspricht,
- Ergänzung der Daten der zweiten Datensätze **durch** Daten des ersten Datensatzes
- Rekonstruktion einer Folge von CT-Bildern, aus den ergänzten zweiten CT-Datensätzen

7. Computerprogramm für eine Steuereinheit (7) zur Steuerung einer Strahlenquelle (S), einer Detektoreinheit (16), einer Antriebsanordnung und einer Rekonstruktionseinheit (10) eines Computertomographen zur Durchführung des Verfahrens nach Anspruch 1 gemäß folgendem Ablauf:
- Erzeugen eines kegelförmigen, einen Untersuchungsbereich (13) bzw. ein darin befindliches Objekt durchsetzenden Strahlenbündels (4) mit einer Strahlenquelle (S),
- Akquisition wenigstens eines ersten, vollständigen CT-Datensatzes,
- Akquisition einer Folge von zweiten CT-Datensätzen, wobei die Relativbewegung zwischen dem Strahlenbündel und dem Objekt jeweils einer geschlossenen - vorzugsweise kreisförmigen - Bahn entspricht,
- Ergänzung der Daten der zweiten Datensätze durch Daten des ersten Datensatzes und
- Rekonstruktion einer Folge von CT-Bildern, aus den ergänzten zweiten CT-Datensätzen.
